(19) Europäisches Patentamt
European Patent Office
Office européen des brevets

(11) EP 3 222 321 A1

(12) EUROPEAN PATENT APPLICATION
published in accordance with Art. 153(4) EPC

(43) Date of publication:
27.09.2017 Bulletin 2017/39

(51) Int Cl.:
A61N 5/10 (2006.01)          E04H 3/08 (2006.01)
G06F 17/50 (2006.01)

(21) Application number: 14906543.5

(22) Date of filing: 21.11.2014

(86) International application number:
PCT/JP2014/080899

(87) International publication number:
WO 2016/079864 (26.05.2016 Gazette 2016/21)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Mitsubishi Electric Corporation
Chiyoda-ku
Tokyo 100-8310 (JP)

(72) Inventor: IWATA Takaaki
Tokyo 100-8310 (JP)

(74) Representative: Sajda, Wolf E.
Meissner Bolte Patentanwälte
Rechtsanwälte Partnerschaft mbB
Postfach 86 06 24
81633 München (DE)

(54) **METHOD FOR SUPPORTING DESIGN OF FACILITY FOR PARTICLE RADIOTHERAPY, METHOD FOR CONSTRUCTING FACILITY FOR PARTICLE RADIOTHERAPY, AND FACILITY FOR PARTICLE RADIOTHERAPY**

(57)     An object is to provide a method of assisting designing of a particle beam therapy facility in which a layout of multiple treatment rooms is well thought out, and a method of constructing the particle beam therapy facility. The method of assisting designing of a particle beam therapy facility of this invention includes: a local-concave region calculation step of calculating a volume of a local concave region which is a concave region between two treatment-room models arranged most adjacent to each other, among multiple treatment-room models arranged in a model space corresponding to a target space for arrangement, or a projected area of the local concave region; a concave-region calculation-result display step of displaying the volume or the projected area of the local concave region calculated in the local-concave region calculation step, on a display device of a design assisting device; and a treatment-room model displacement step of displacing the treatment-room model in the model space in response to a displacement instruction for that treatment-room model, when no operation- termination instruction is issued after the concave-region calculation-result display step; wherein the local-concave region calculation step, the concave-region calculation-result display step and treatment-room model displacement step are repeated until an operation-termination instruction is issued.

FIG. 1

**Description**

TECHNICAL FIELD

The present invention relates to a particle beam therapy facility that is provided with a particle beam therapy system and a method of constructing the particle beam therapy facility and, in particular, relates to a particle beam therapy facility in which a layout of multiple treatment rooms is well thought out.

BACKGROUND ART

Particle beam therapy systems are quite large systems, so that, in particular in Japan whose national land is small, how to downsize the system itself and how to reduce the ground-floor area of its installation place have been thought out.

In Patent Document 1, there is described a charged- particle beam irradiation apparatus (particle beam therapy system) that is provided with: plural second transport lines for transporting a charged particle ray (charged particle beam) to respective irradiation rooms; and a line switching means by which the second transport line for a guiding destination can be selectively switched.

The charged-particle beam irradiation apparatus of Patent Document 1 is provided with: a first transport line (common transport line) for transporting the charged particle beam sent out from an accelerator; and the plural second transport lines (individual transport lines) each provided for each of the multiple irradiation rooms, for further transporting the charged particle beam transported by the first transport line, to each of the irradiation rooms; wherein the line switching means is provided between the first transport line and the second transport lines, by which the charged particle beam from the first transport line is guided to any one of the second transport lines and the second transport line for a guiding destination can be selectively switched.

The multiple irradiation rooms are arranged radially around the line switching means, and the line switching means includes an electromagnet for guiding the charged particle beam and a rotation mechanism for rotating the electromagnet, and switches between the second transport lines for respective guiding destinations by rotating the electromagnet.

Meanwhile, in Patent Document 2, there is described an accelerated particle irradiation facility in which plural irradiation apparatuses are placed in a manner horizontally shifted to each other, and are each placed on a floor different to a floor on which a particle accelerator is placed. A guide line in the accelerated particle irradiation facility of Patent Document 2 includes an extraction passage that is connected to and extending horizontally from the particle accelerator and is then bent into the vertical direction.

At a position after passing through the extraction passage, the guide line branches into two lines so that angles of 0° and 90°, angles of 45° and -45°, or angles of 45° and 135° are established in planer view, respectively between the line before branching and the two lines after branching, thus providing an angle of 90° in a horizontal plane as the difference between a connection direction toward one irradiation apparatus and a connection direction toward the other irradiation apparatus.

LIST OF CITATIONS

PATENT DOCUMENTS

Patent Document 1:    Japanese Patent Application Laid-Open JP 2012-100 915 A (Para.[0006], Para.[0022] to [0034], FIG. 1, FIG. 2)
Patent Document 2:    Japanese Patent JP 5 526 166 B2 (Para.[0007], Para. [0056] to [0061], FIG. 15, FIG. 16)

SUMMARY OF THE INVENTION

PROBLEMS TO BE SOLVED BY THE INVENTION

It is acknowledged that the ground-floor area of the installation place for the particle beam therapy system is reduced to some extent when, as described in Patent Document 1, the multiple irradiation rooms are arranged radially or when, as described in Patent Document 2, the angles of 45° and -45° or the like by which an angle of 90° is provided therebetween are established in planar view, respectively between the line before branching and the two lines after branching.

However, actually in many cases, the installation place that the hospital side can get ready for the particle beam therapy system is restricted, so that the shape and the ground-floor area of the installation place are given in an unintentional manner. Meanwhile, when, as described in Patent Document 2, the angles of 45° and -45° or the like by which an angle of 90° is provided therebetween are established in planar view, respectively between the line before branching and the two lines after branching, whether this provides an efficient layout or not depends on the shape and size of a rotary gantry and the shape and size of the installation place.

The rotary gantry is a rotatable device for radiating the charged particle beam to a patient from an arbitrary direction, and, though depending on the shape and size of the rotary gantry and the shape and size of the installation place, inherently, there may be cases where such an arrangement can not be achieved that establishes the angles of 45° and -45° or the like by which an angle of 90° is provided therebetween.

As described above, according to the heretofore- proposed techniques, although stereotypical handling methods are shown that reduce, to some extent, the ground-floor area of the installation place for the particle beam therapy system, there is a problem that it is unable to differentially make handling in consideration of the shape and size of the rotary gantry and the shape and size of the installation place.

This invention has been made in view of the above problem, and an object thereof is to provide a method of assisting designing of a particle beam therapy facility in which the layout of the multiple treatment rooms is well thought out and a method of constructing the particle beam therapy facility, by taking into consideration the shapes and sizes of the rotary gantry and the treatment room, such as a gantry room, including a treatment region adjacent to the rotary gantry, and the shape and size of the installation place for the treatment rooms.

## MEANS FOR SOLVING THE PROBLEMS

A method of assisting designing of a particle beam therapy facility according to the invention comprises:

a treatment- room model preparation step of preparing a treatment-room model that is a three-dimensional model of each of the treatment rooms;
a treatment-room model arrangement step of arranging the multiple treatment-room models, at initial positions in a model space corresponding to a target space for arrangement;
a local-concave region calculation step of calculating: a volume of a local concave region which is a concave region between two treatment-room models among the multiple treatment-room models, that are arranged most adjacent to each other; or a projected area which is developed when the local concave region is two-dimensionally projected toward a floor; a concave-region calculation-result display step of displaying the volume or projected area of the local concave region calculated in the local-concave region calculation step, on a display device of a design assisting device; and
a treatment-room model displacement step of displacing the treatment-room model in the model space in response to a displacement instruction for that treatment-room model, when an operation-termination instruction is not issued after the concave-region calculation-result display step; wherein the local-concave region calculation step, the concave-region calculation-result display step and the treatment-room model displacement step are repeated until the operation- termination instruction is issued.

The local concave region according to the invention is a region composed of a set of points on lines that connect to each other, mutually-facing outer peripheries of the two treatment-room models arranged most adjacent, or, in the case where a shield wall is placed between the two treatment-room models arranged most adjacent, a region composed of a set of object points that are points on lines that connect to each other, mutually-facing outer peripheries of the two treatment-room models in that case, except for points in the shield wall.

## EFFECT OF THE INVENTION

By the method of assisting designing of a particle beam therapy facility according to the invention, the volume or projected area of the concave region between two treatment-room models among the multiple treatment-room models, that are arranged most adjacent to each other, is calculated and displayed on the display device. This allows the calculation result of the area or volume of the concave region to be displayed according to the arranged positions of the treatment-room models, so that the layout of the multiple treatment rooms can be optimized so as to reduce the concave region as much as possible.

## BRIEF DESCRIPTION OF THE DRAWINGS

FIG. 1     is a flowchart showing a method of assisting designing of a particle beam therapy facility, according to Embodiment 1 of the invention.

FIG. 2     is a diagram showing a treatment-room model of the invention.

FIG. 3     is a diagram showing a display example of arrangement of treatment-room models, according to FIG. 1.

FIG. 4     is a diagram showing a display example of calculation result of a volume or projected area of a local concave region, according to FIG. 1.

FIG. 5     is a schematic configuration diagram of a particle beam therapy system that is arranged in a particle beam therapy facility of the invention.

FIG. 6     is a diagram showing a configuration of a particle beam irradiation apparatus shown in FIG. 5.

FIG. 7     is a diagram illustrating a concave region between two treatment-room models.

FIG. 8     is a diagram showing an example in which two treatment- room models are optimally arranged.

FIG. 9     is a diagram showing an example in which two treatment-room models are optimally arranged in a target space for arrangement.

FIG. 10     is a diagram showing an example of a particle beam therapy facility according to Embodiment 1 of the invention.

FIG. 11     is a diagram showing another example of the particle beam therapy facility according to Embodiment 1 of the invention.

FIG. 12     is a flowchart showing a method of assisting designing of a particle beam therapy facility, according to Embodiment 2 of the invention.

FIG. 13     is a flowchart showing a method of assisting designing of a particle beam therapy facility, according to Embodiment 3 of the invention.

FIG. 14     is a diagram showing a display example of calculation result of volumes or projected areas of a local concave region and a residual-space concave region, according to FIG. 13.

FIG. 15     is a diagram illustrating a concave region with respect to a residual space of a particle beam therapy facility.

FIG. 16     is a diagram illustrating another concave region with respect to a residual space of a particle beam therapy facility.

FIG. 17     is a flowchart showing a method of assisting designing of a particle beam therapy facility, according to Embodiment 4 of the invention.

FIG. 18     is a flowchart showing a method of constructing a particle beam therapy facility, according to Embodiment 5 of the invention.

FIG. 19     is a diagram showing an example in which two treatment- room models and an accelerator model are optimally arranged in a target space for arrangement.

FIG. 20     is a diagram showing an example of a particle beam therapy facility according to Embodiment 5 of the invention.

MODES FOR CARRYING OUT THE INVENTION

Embodiment 1

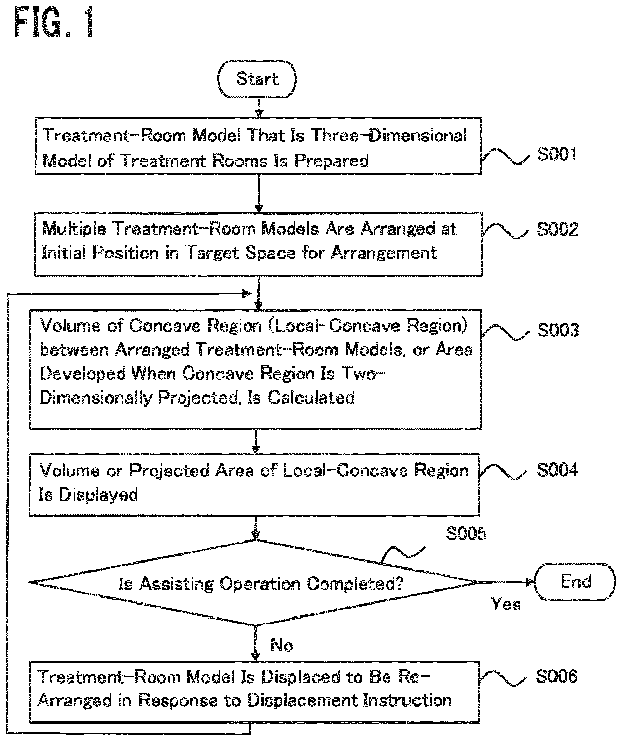

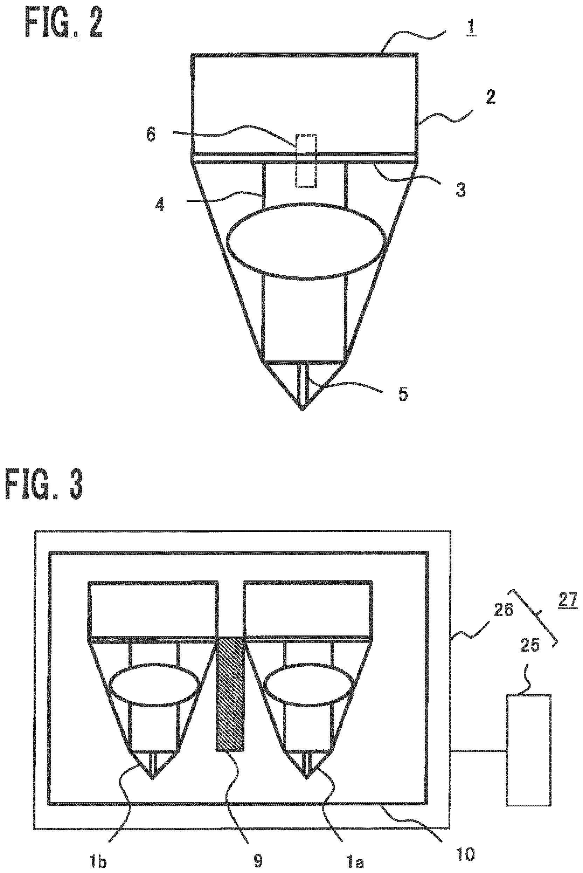

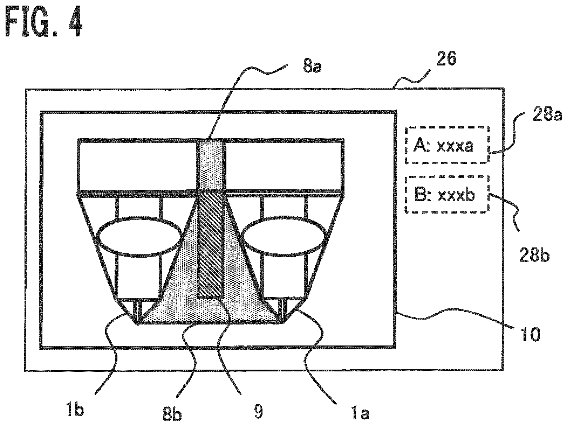

FIG. 1 is a flowchart showing a method of assisting designing of a particle beam therapy facility, according to Embodiment 1 of the invention. FIG. 2 is a diagram showing a treatment-room model of the invention. FIG. 3 is a diagram showing a display example of arrangement of treatment-room models, according to FIG. 1, and FIG. 4 is a diagram showing a display example of calculation result of a volume or projected area of a local concave region, according to FIG. 1.

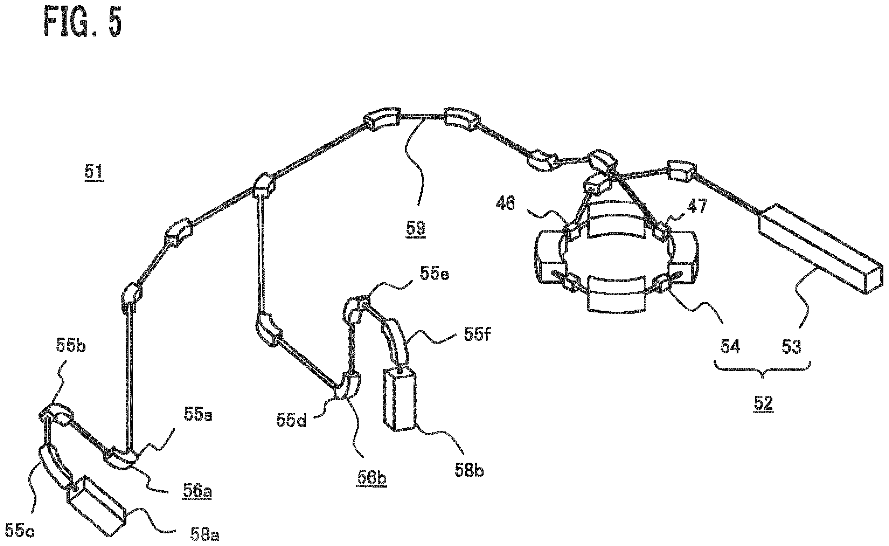

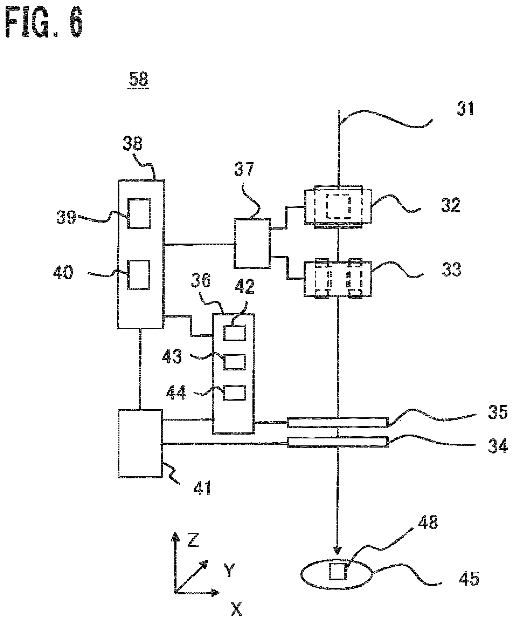

FIG. 5 is a schematic configuration diagram of a particle beam therapy system that is arranged in a particle beam therapy facility of the invention, and FIG. 6 is a diagram showing a configuration of a particle beam irradiation apparatus shown in FIG. 5. First, an outline of a particle beam therapy system that is arranged in a particle beam therapy facility will be described, and thereafter, the method of assisting designing of a particle beam therapy facility will be described.

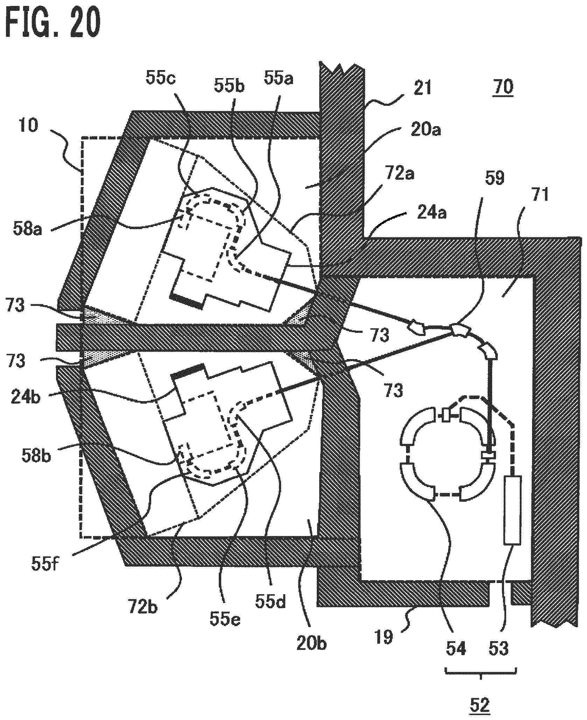

In FIG. 5, a particle beam therapy system 51 includes a beam generation apparatus 52, a beam transport system 59 and particle beam irradiation apparatuses 58a, 58b. The beam generation apparatus 52 includes an ion source (not shown), a pre-accelerator 53 and an accelerator 54. The particle beam irradiation apparatus 58a is placed in a rotary gantry 24a (see, FIG. 20) in a gantry room 20a. The particle beam irradiation apparatus 58b is placed in a rotary gantry 24b (see, FIG. 20) in a gantry room 20b. The gantry rooms 20a, 20b are treatment rooms in which the rotary gantries 24a, 24b are placed.

The role of the beam transport system 59 is to communicate between the accelerator 54 and the particle beam irradiation apparatuses 58a, 58b. The beam transport system 59 is partly placed in the rotary gantry 24a and is provided with, at that part, a plurality of bending electromagnets 55a, 55b, 55c. The beam transport system 59 is partly placed in the rotary gantry 24b and is provided with, at that part, a plurality of bending electromagnets 55d, 55e, 55f. The part of the beam transport system 59 placed in the rotary gantry 24a is a rotary-gantry mounting portion 56a, and the part of the beam transport system 59 placed in the rotary gantry 24b is a rotary-gantry mounting portion 56b.

The charged particle beam generated by the ion source, that is a particle beam such as a proton beam, etc. is accelerated by the pre-accelerator 53 and injected into the accelerator 54 through an injection device 46. The accelerator 54 is a synchrotron, for example. The charged particle beam is accelerated up to a given energy. The charged particle beam emitted from an emission device 47 of the accelerator 54, is transported through the beam transport system 59 to the particle beam irradiation apparatuses 58a, 58b.

The particle beam irradiation apparatuses 58a, 58b each radiate the charged particle beam to a diseased site (irradiation target) 48 of a patient 45 (see, FIG. 6). For the particle beam irradiation apparatuses, numeral 58 is used collectively, and numerals 58a, 58b are used when they are to be described distinctively.

The charged particle beam 31 generated by the beam generation apparatus 52 and accelerated up to the given energy, is brought through the beam transport system 59 to the particle beam irradiation apparatus 58. In FIG. 6, the particle beam irradiation apparatus 58 includes: an X-direction scanning electromagnet 32 and a Y-direction scanning electromagnet 33 which scan the charged particle beam 31, respectively in an X-direction and a Y-direction that are directions perpendicular to the charged particle beam 31; a position monitor 34; a dose monitor 35; a dose-data converter 36; a beam-data processing device 41; a scanning-electromagnet power source 37; and an irradiation management device 38 for controlling the particle beam irradiation apparatus 58. The irradiation management device 38 includes an irradiation control computer 39 and an irradiation control device 40. The dose-data converter 36 includes a trigger generation unit 42, a spot counter 43 and an inter-spot counter 44. Note that in FIG. 6, the travelling direction of the charged particle beam 31 is a direction of -Z.

The X-direction scanning electromagnet 32 is a scanning electromagnet for scanning the charged particle beam 31 in the X-direction, and the Y-direction scanning electromagnet 33 is a scanning electromagnet for scanning the charged particle beam 31 in the Y-direction. With respect to the charged particle beam 31 scanned by the X-direction scanning electromagnet 32 and the Y-direction scanning electromagnet 33, the position monitor 34 detects beam information for calculating a passing position (gravity center position) and a size of the beam that passes therethrough.

The beam-data processing device 41 calculates the passing position (gravity center position) and the size of the charged particle beam 31 on the basis of the beam information that comprises a plurality of analog signals detected by the position monitor 34. Further, the beam-data processing device 41 generates an abnormality detection signal indicative of a position abnormality and/or a size abnormality of the charged particle beam 31, and outputs the abnormality detection signal to the irradiation management device 38.

The dose monitor 35 detects the dose of the charged particle beam 31. The irradiation management device 38 controls the irradiation position of the charged particle beam 31 in the diseased site 48 of the patient 45 on the basis of treatment plan data prepared by a treatment plan device (not shown), and moves the charged particle beam 31 to a next irradiation position when the dose having been measured by the dose monitor 35 and converted by the dose-data converter 36 into digital data, reaches a target dose.

The scanning-electromagnet power source 37 changes setup currents for the X-direction scanning electromagnet 32 and the Y-direction scanning electromagnet 33 on the basis of control inputs (commands) output from the irradiation management device 38 for the X-direction scanning electromagnet 32 and the Y-direction scanning electromagnet 33.

Here, the scanning irradiation method of the particle beam irradiation apparatus 58 is assumed to be a raster-scanning irradiation method in which the charged particle beam 31 is not stopped when the irradiation position of the charged particle beam 31 is changed, and is a method in which the beam irradiation position moves between spot positions successively like a spot-scanning irradiation method. The spot counter 43 serves to measure an irradiation dose during when the beam irradiation position of the charged particle beam 31 is staying.

The inter-spot counter 44 serves to measure an irradiation dose during when the beam irradiation position of the charged particle beam 31 is moving. The trigger generation unit 42 serves to generate a dose completion signal when the dose of the charged particle beam 31 at a beam irradiation position reaches the target irradiation dose.

Next, the method of assisting designing of a particle beam therapy facility will be described. The method of assisting designing of a particle beam therapy facility is executed by a computer 25, and an intermediate situation and a calculation result, during design assisting operation, are displayed on a display device 26. As shown in FIG. 3, a design assisting device 27 for executing the method of assisting designing of a particle beam therapy facility includes the computer 25 and the display device 26.

In Step S001 in FIG. 1, a treatment-room model that is a three-dimensional model of each of the treatment rooms is prepared (treatment-room model preparation step). In FIG. 2, an example of the treatment-room model is shown. The treatment-room model includes at least a boundary for distinguishing it from another region. A gantry-room model 1 that is the treatment-room model includes, for example, a gantry body 4, a gantry front panel 3, a vacuum duct 5 and an open-space region 2 connected to an inner region of the gantry body 4.

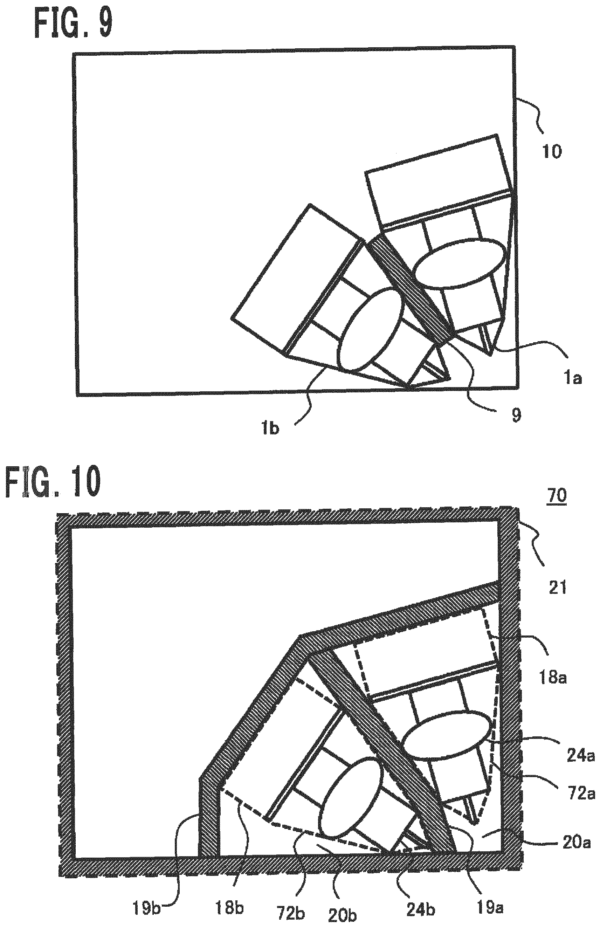

At the time of treatment, a treatment table 6 on which the patient 45 is laid is placed astride between an inner region corresponding to the gantry body 4 in the rotary gantry 24a or 24b and an open-space region 18a or 18b (see, FIG. 10) corresponding to the open-space region 2. The vacuum duct 5 is a vacuum duct of the beam transport system 59. It is noted that, in the diagram in which the gantry-room model 1 is illustrated, the gantry-room model 1 is basically shown as a top view viewed from the upper side.

In Step S002, the multiple treatment-room models are arranged at initial positions inside an arrangement-region frame 10 which represents a boundary of a model space that is a modeled target space for arrangement (treatment-room model arrangement step). A space that is partitioned with the arrangement-region frame 10 is the model space corresponding to the target space for arrangement. As shown in FIG. 3, the positions of two gantry-room models 1a, 1b are the initial positions of the treatment-room models, for example.

According to the initial positions of the treatment- room models, the two gantry-room models 1a, 1b are arranged adjacent to each other at a distance corresponding to the thickness of a shield wall 9. In this manner, according to the initial positions of the treatment-room models, the multiple gantry-room models 1a, 1b are arranged without positionally interfering with each other. The result from execution of the treatment-room model arrangement step is displayed as shown in FIG. 3 on the display device 26.

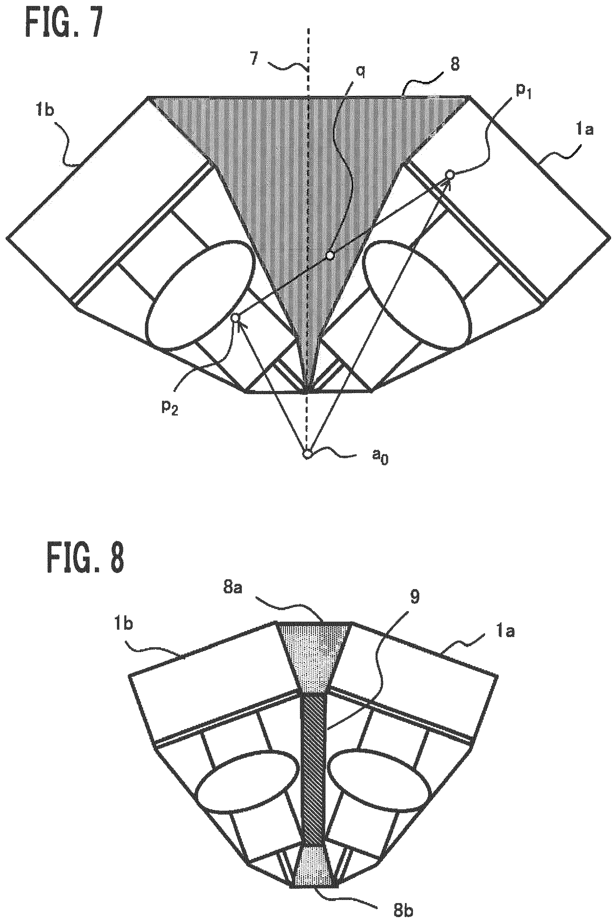

In Step S003, a volume of a concave region 8 (see, FIG. 7) between the thus-arranged treatment-room models (gantry-room models 1a, 1b), or an area (projected area) developed when the concave region is two-dimensionally projected toward a floor, is calculated (local-concave region calculation step). In Step S004, the volume or projected area calculated in the local-concave region calculation step is displayed on the display device 26 (concave-region calculation-result display step).

The concave region 8 between the treatment-room models (gantry-room models 1a, 1b) is, where appropriate, referred also to as a local concave region 8 in order to distinguish it from a concave region 15 to be described later. The local-concave region calculation step is executed when, for example, a calculation start command is input through an input device, such as a keyboard, a mouse, etc. of the computer 25.

As shown in FIG. 4, the result from execution of the local-concave region calculation step and the concave-region calculation-result display step is displayed on the display device 26. In FIG. 4, there are shown two concave regions, in which a concave region 8a is illustrated in the side of the open-space region 2 and a concave region 8b is illustrated in the side of the gantry body 4. A concave-region indication 28a represents the volume or projected area of the concave region 8a, and a concave-region indication 28b represents the volume or projected area of the concave region 8a.

In Step S005, whether the assisting operation required from an operator is completed or not is judged, so that the operation is terminated when an assisting-operation-termination instruction is issued, or the flow moves to Step S006 when no assisting- operation-termination instruction is issued.

In Step S006, in response to a displacement instruction by the operator, the treatment-room model is displaced to be re-arranged (treatment-room model displacement step). After execution of Step S006, the flow returns to Step S003, so that the assisting operation is continued.

Using FIG. 7, description will be made about the concave region (local concave region). FIG. 7 is a diagram illustrating the concave region(s) between two treatment-room models. For the concave regions (local concave regions), the numeral 8 is used collectively, and the numerals 8a, 8b are used when they are to be described distinctively. In FIG. 7, an example is shown in which the gantry-room models 1a, 1b are arranged at angles of +45° and -45°, respectively, with respect to a center line 7. The concave region 8 is defined as follows.

The concave region 8 is a region composed of a set of points q. Points in the gantry-room models 1a, 1b are assumed to be a set p. The set p can be represented as a Formula (1).

$$p \mid G \in p \qquad \ldots(1)$$

where, G represents a set area of the gantry-room models 1a, 1b.

When any two points in the set area G are defined as $p_1$ and $p_2$, the set q can be represented as a Formula (2). Namely, the set q is a set that satisfies Condition 1, Condition 2 and Condition 3.

$$q \mid \text{Condition 1, Condition 2, Condition 3} \quad \ldots(2),$$

where, Condition 1, Condition 2 and Condition 3 are represented by a Formula (3), a Formula (4) and a Formula (5), respectively.

$$q = \lambda p_1 + (1 - \lambda)p_2 \qquad \ldots(3)$$

$$0 < \lambda < 1 \qquad \ldots (4).$$

[Mathematical Expression 1]

$$q \notin G \qquad \cdots (5)$$

Note that $\lambda$ denotes an actual number.

In FIG. 7, a reference point $a_0$ for the points $p_1$, $p_2$ is indicated on the center line 7. Further, the points $p_1$, $p_2$ are represented by vectors from the reference point $a_0$.

When the shield wall 9 is placed, the concave region 8 is given as a region that excludes the shield wall 9. For example, in FIG. 4, the two concave regions 8a, 8b are indicated in which the shield wall 9 is not included. Note that, when a set area of the shield wall 9 is defined as W, such a set q that satisfies a Formula (6) resulting from adding Condition 4 to the Formula (2), corresponds to the concave regions 8a, 8b shown in FIG. 4.

$$q \mid \text{Condition 1, Condition 2, Condition 3, Condition 4} \qquad \ldots (6)$$

where, Condition 4 is represented by a Formula (7).

[Mathematical Expression 2]

$$q \notin W \qquad \cdots (7)$$

Although the concave region 8 is defined as described above, it can be defined in other words as follows: when such points on lines that connect to each other, mutually-facing respective points (outer periphery points) on outer peripheries of the gantry-room models 1a, 1b that are adjacent to each other, except for points in the shield wall 9, are defined as object points, the concave region 8 is a region composed of these object points.

FIG. 8 is a diagram showing an example in which two treatment-room models are optimally arranged. In FIG. 8, the shield wall 9 is placed in contact with the adjacent gantry-room models 1a, 1b at their outer peripheries in the side of the gantry body 4. In FIG. 8, the projected area of the concave regions between the treatment-room models, namely, the projected area of a total region of the concave regions 8a, 8b, is equal to or less than one fourth of the projected area of the gantry-room model that is the gantry- room model 1a or the gantry-room model 1b.

FIG. 9 is a diagram showing an example in which two treatment-room models are optimally arranged in a target space for arrangement. In FIG. 9, the gantry-room models 1a, 1b are arranged at a right corner in the arrangement-region frame 10 while keeping the relative positions of the gantry-room models 1a, 1b shown in FIG. 8. FIG. 9 shows an arrangement after completion of the operation by the flowchart in FIG. 1.

A particle beam therapy facility 70 constructed based on the arrangement of FIG. 9 is shown in FIG. 10. FIG. 10 is a diagram showing an example of the particle beam therapy facility according to Embodiment 1 of the invention. In the particle beam therapy facility 70 of FIG. 10, two gantry rooms 20a, 20b and shield walls 19a, 19b are arranged in a region surrounded by a building wall 21. The arrangement-region frame 10 in FIG. 9 corresponds to a boundary around the region surrounded by the building wall 21. In FIG. 10, model-corresponding frames 72a, 72b which correspond to the outer peripheries (boundaries) of the gantry-room models 1a, 1b in FIG. 9, are shown by broken lines.

The rotary gantries 24a, 24b are real rotary gantries in the gantry-room models 1a, 1b, each provided with the gantry body 4 and the gantry front panel 3. The open-space regions 18a, 18b are space regions corresponding to the open-space regions 2 of the gantry-room models 1a, 1b. In the particle beam therapy facility 70 of FIG. 10, the relative positions of the two gantry rooms 20a, 20b is the same as the relative positions of the gantry-room models 1a, 1b in FIG. 9.

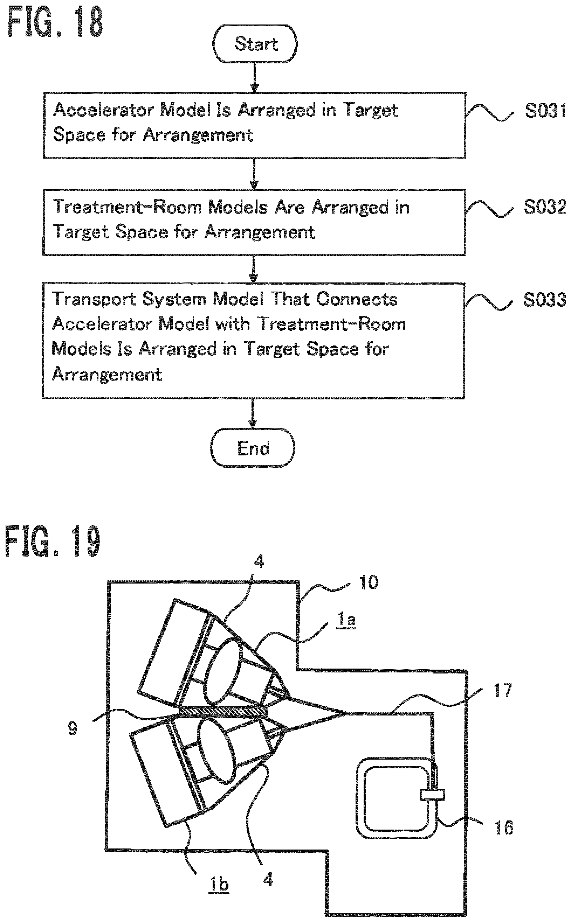

Using the method of assisting designing of a particle beam therapy facility of Embodiment 1 makes it possible to properly arrange the gantry-room models 1a, 1b in the arrangement-region frame 10. Thus, the layout of the multiple treatment rooms (gantry rooms 20a, 20b) can be optimized so as to reduce, as much as possible, the concave region 8 which is a region that is essentially unnecessary and useless. In FIG. 9, the arrangement-region frame 10 is exemplified by a rectangle shape; however, the layout shall be studied using the arrangement-region frame 10 with a shape matched to the configuration of the building (see, FIG. 19) in which the gantry rooms 20a, 20b are to be placed and thus, for the arrangement-region frame, the shape and the size of each of the treatment rooms, such as the gantry rooms 20a, 20b, etc., and the shape and the size of the installation place for the treatment rooms may be taken into consideration.

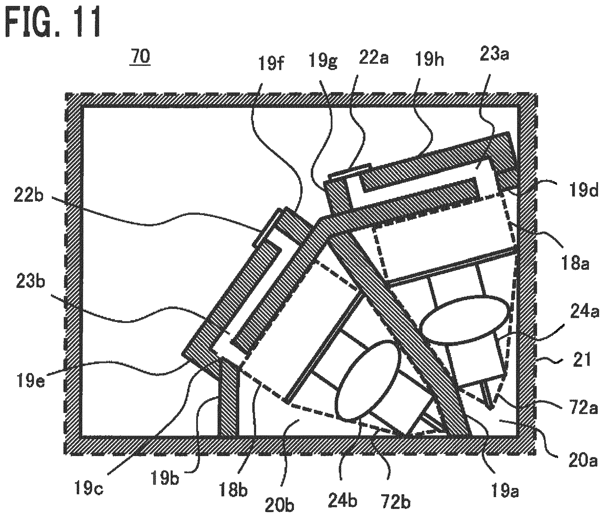

FIG. 11 is a diagram showing another example of the particle beam therapy facility according to Embodiment 1 of the invention. The particle beam therapy facility 70 of FIG. 11 is an example in which labyrinth passages 23a, 23b are formed in the gantry rooms 20a, 20b. In FIG. 11, on the side of the gantry room 20a where the open-space region 18a is placed and on the side of the gantry room 20b where the open-space region 18b is placed, shield walls 19b, 19c, 19d, 19e, 19f, 19g, 19h that form the labyrinth passages 23 a, 23b are composed, and doors 22a, 22b are arranged respectively at the entrances of the labyrinth passages 23a, 23b.

It is noted that, in the case where the number of the multiple treatment rooms is three or more, among three or more treatment-room models, each two of the treatment-room models that are arranged most adjacent to each other is determined as a target pair, and the local-concave region calculation step is executed for each target pair. Then, in the concave-region calculation-result display step, the volume of the local-concave region 8 or the projected area of the local-concave region 8 calculated for each target pair is displayed on the display device 26.

The method of assisting designing of a particle beam therapy facility of Embodiment 1 comprises; the treatment-room model preparation step of preparing the treatment-room model (gantry-room model 1) that is a three-dimensional model of each of the treatment rooms (gantry rooms 20a, 20b); the treatment-room model arrangement step of arranging the multiple treatment-room models (gantry-room models 1a, 1b) at initial positions in the model space corresponding to the target space for arrangement; the local-concave region calculation step of calculating, a volume of the local concave region 8 which is a concave region between two treatment-room models (gantry-room models 1a, 1b) among the multiple treatment-room models (gantry-room models 1a, 1b), that are arranged most adjacent to each other, or a projected area which is developed when the local concave region 8 is two-dimensionally projected toward a floor; the concave- region calculation-result display step of displaying the volume or projected region of the local concave region 8 calculated in the local-concave region calculation step, on the display device 26 of the design assisting device 27; and the treatment-room model displacement step of displacing the treatment-room model (gantry-room models 1a, 1b) in the model space in response to a displacement instruction for that treatment-room model (gantry-room models 1a, 1b), when no operation-termination instruction is issued after the concave-region calculation-result display step; wherein the local-concave region calculation step, the concave-region calculation-result display step and the treatment-room model displacement step are repeated until an operation-termination instruction is issued.

The local concave region 8 according to the method of assisting designing of a particle beam therapy facility of Embodiment 1 is a region composed of a set of points on lines that connect to each other, the mutually-facing outer peripheries of the two treatment-room models (gantry-room models 1a, 1b) arranged most adjacent, or, in the case where the shield wall 9 is placed between the two treatment-room models (gantry-room models 1a, 1b) arranged most adjacent, a region composed of a set of object points that are points on lines that connect to each other the mutually-facing outer peripheries of the two treatment-room models (gantry-room models 1a, 1b) in that case, except for points in the shield wall 9.

Because of this configuration, according to the method of assisting designing of a particle beam therapy facility of Embodiment 1, the volume or projected area of the concave region 8 between the two treatment-room models (gantry-room models 1 a, 1b) among the multiple treatment-room models (gantry-room models 1a, 1b), that are arranged most adjacent to each other, is calculated and displayed on the display device 26. This allows the calculation result of the area or volume of the concave region 8 to be displayed according to the arranged positions of the treatment-room models (gantry-room models 1a, 1b), so that the layout of the multiple treatment rooms (gantry rooms 20a, 20b) can be optimized so as to reduce the concave region 8 as much as possible.

Embodiment 2

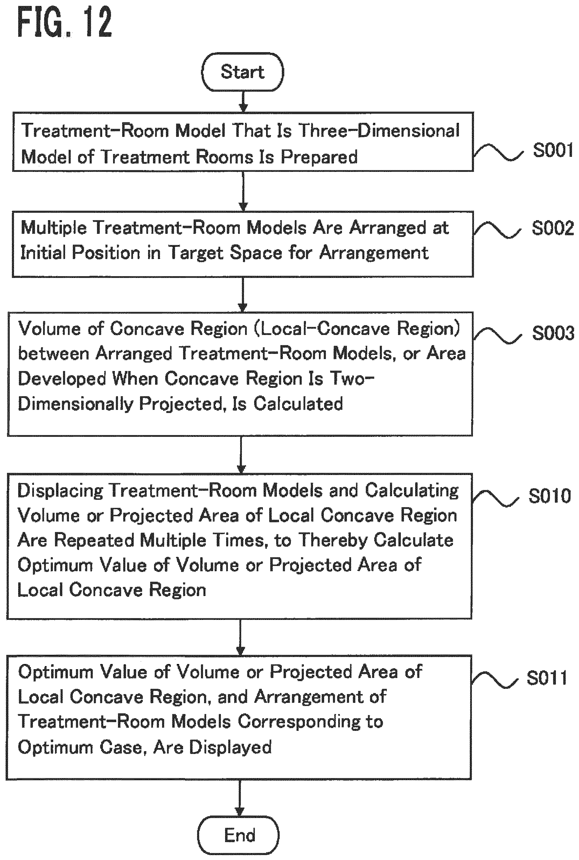

FIG. 12 is a flowchart showing a method of assisting designing of a particle beam therapy facility, according to Embodiment 2 of the invention. In Embodiment 1, an example is shown in which the gantry-room model 1 is displaced according to an operator's instruction, and at each displacement, the volume or projected area of the concave region 8 is calculated, whereas what is shown in Embodiment 2 is an example in which its optimum value is calculated through recursive calculation by the computer 25. The flowchart of FIG. 12 differs from that of FIG. 1 in that Step S010 and Step S011 are added in place of Steps S004, S005 and S006 in FIG. 1.

Steps S010 and S011 that are steps different to those in FIG. 1 will be described. In Step S010, the computer 25 repeats multiple times, displacing the gantry-room models 1a, 1b that are the treatment-room models and calculating the volume or projected area of the local concave region 8, to thereby calculate an optimum value of the volume or projected area of the local concave region 8 (optimum-value calculation step). As a criterion for determining whether the volume or projected area of the local concave region 8 is its optimum value or not, the fact is, for example, used that the value of the volume or projected area of the local concave region 8 becomes minimum in the arrangement-region frame 10. For example, at the time of recursive calculation of the volume or projected area of the local concave region 8, the next position of each treatment-room model is determined using a steepest descent method or the like.

In Step S011, the optimum value of the volume or projected area of the local concave region 8 calculated in Step S010, and the arrangement of the gantry-room models 1a, 1b that are the treatment-room models from which the optimum value has been calculated, are displayed on the display device 26 (optimum-value calculation-result display step).

It is noted that, when plural similar optimum values (suboptimum values) are present in Step S010, each suboptimum value of the volume or projected area of the local concave area 8 corresponding to the respective extracted suboptimum values, and each arrangement of the gantry-room models 1a, 1b that are the treatment-room models from which the suboptimum values have been calculated, are displayed on the display device 26 in Step S011.

According to the method of assisting designing of a particle beam therapy facility of Embodiment 2, the computer 25 calculates, through recursive calculation, an optimum value of the volume or projected area of the concave region 8, so that the layout of the multiple treatment rooms (gantry rooms 20a, 20b) can be optimized so as to reduce the concave region 8 as much as possible, faster than in the case of Embodiment 1. Further, according to the method of assisting designing of a particle beam therapy facility of Embodiment 2, the layout of the multiple treatment rooms (gantry rooms 20a, 20b) can be optimized more efficiently than in the case of Embodiment 1.

It is noted that, in the case where the number of the multiple treatment rooms is three or more, it suffices: to determine, among three or more treatment-room models, each two of the treatment- room models that are arranged most adjacent to each other, as a target pair; to execute the local-concave region calculation step for each target pair; and to repetitively perform in the optimum-value calculation step, multiple times for each target pair, displacing the treatment-room model and calculating the volume of the local concave region 8 or the projected area of the local concave region 8, to thereby calculate the optimum value.

The method of assisting designing of a particle beam therapy facility of Embodiment 2 is characterized by comprising: the treatment-room model preparation step of preparing the treatment-room model (gantry-room model 1) that is a three-dimensional model of each of the treatment rooms (gantry rooms 20a, 20b); the treatment-room model arrangement step of arranging the multiple treatment-room models (gantry-room models 1a, 1b) at initial positions in the model space corresponding to the target space for arrangement; the local-concave region calculation step of determining, among the multiple treatment-room models (gantry-room models 1a, 1b), each two of said treatment-room models (gantry-room models 1a, 1b), that are arranged most adjacent to each other, as a target pair, and calculating, for each target pair, a volume of the local concave region 8 which is a concave region between two said treatment-room models (gantry-room models 1a, 1b) in the target pair, or a projected area which is developed when the local concave region 8 is two-dimensionally projected toward a floor; the optimum-value calculation step of repetitively performing, multiple times for each target pair, displacing the treatment-room model (gantry-room models 1a, 1b) by use of the design assisting device 27 and calculating the volume of the local concave region 8 or the projected area of the local concave region 8, to thereby calculate an optimum value thereof; and the optimum-value calculation-result display step of displaying the optimum value of the volume of the local concave region 8 or the optimum value of the projected area that is calculated in the optimum-value calculation step, and an arrangement of the treatment-room models (gantry-room models 1a, 1b) corresponding to the case of that optimum value, on the display device 26 of the design assisting device 27.

Because of this configuration, according to the method of assisting designing of a particle beam therapy facility of Embodiment 2, the optimum value of the volume or projected area of the concave region 8 is calculated through recursive calculation by the computer 25 of the design assisting device 27, so that the layout of the multiple treatment rooms (gantry rooms 20a, 20b) can be optimized so as to reduce the concave region 8 as much as possible, faster than in the case of Embodiment 1. Further, according to the method of assisting designing of a particle beam therapy facility of Embodiment 2, the layout of the multiple treatment rooms (gantry rooms 20a, 20b) can be optimized more efficiently than in the case of Embodiment 1.

Embodiment 3

In Embodiment 1, an example is shown in which the volume or projected area of the local concave region 8 is optimized; however, it is desired that an effectively-usable region be ensured also in a region in the arrangement-region frame 10 where the gantry-room model 1 is not arranged. In Embodiment 3, a method of assisting designing of a particle beam therapy facility will be described which can optimize the region in the arrangement-region frame 10 where the gantry-room model 1 is not arranged.

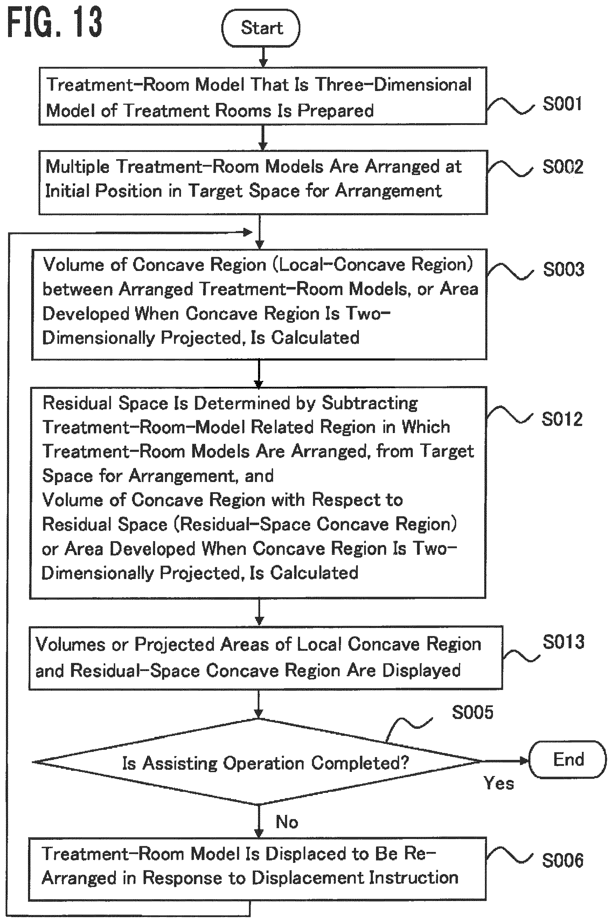

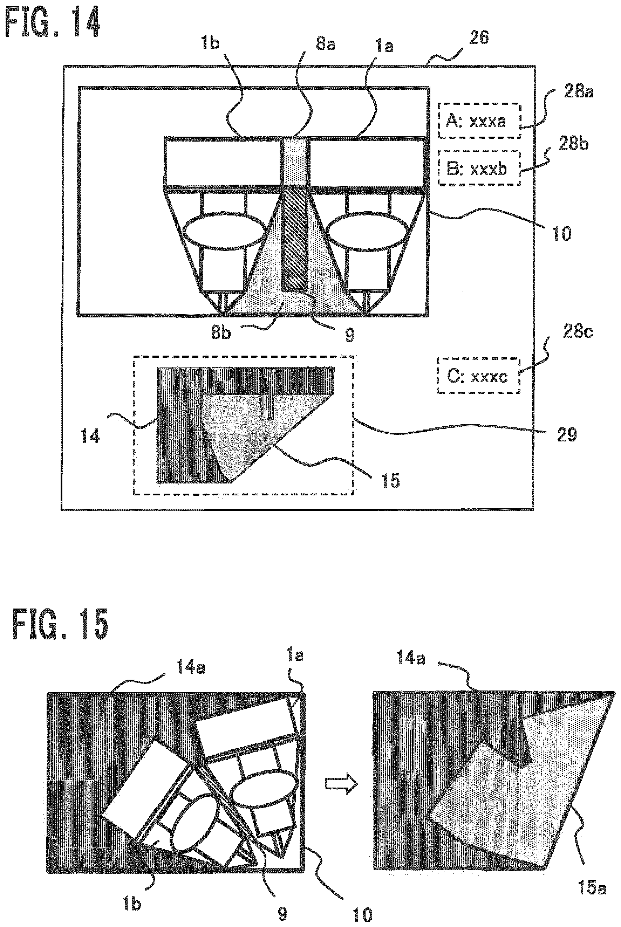

FIG. 13 is a flowchart showing a method of assisting designing of a particle beam therapy facility, according to Embodiment 3 of the invention. FIG. 14 is a diagram showing a display example of calculation result of volumes or projected areas of a local concave region and a residual-space concave region, according to FIG. 13.

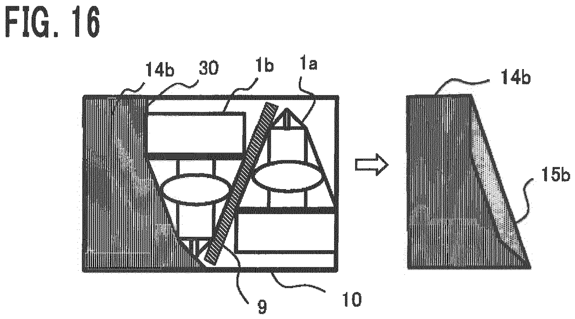

FIG. 15 is a diagram illustrating a concave region with respect to a residual space of a particle beam therapy facility, and FIG. 16 is a diagram illustrating another concave region with respect to a residual space of a particle beam therapy facility. First, using FIG. 15 and FIG. 16, the residual space (three-dimensional region) of the particle beam therapy facility and the concave region (residual-space concave region) with respect to that residual space will be described. The concave region with respect to the residual space is, where appropriate, referred to as a residual-space concave region.

As this region, such a region is assumed that allows an apparatus and a structural part to be arranged therein. Thus, a two-dimensional region shown in a top view in which the gantry rooms 20a, 20b are arranged as in FIG. 10 and a three-dimensional region resulting from expanding the two- dimensional region vertically from the floor to the ceiling, will be considered for the residual space and the residual-space concave region of the particle beam therapy facility.

In FIG. 15 and FIG. 16, shown in the left side of each arrow is a top view in which the gantry-room models 1a, 1b are arranged. Each shown in the right side of an arrow in FIG. 15 and FIG. 16 is an extracted view of the residual space and the residual-space concave region. In FIG. 15, the gantry-room models 1a, 1b are arranged on the right side in the arrangement-region frame 10 so as to be adjacent to each other and in contact with the shield wall 9. Note that the arrow in FIG. 15 indicates that a residual space 14a and a residual-space concave region 15a shown in the right side are extracted from the arrangement information about the gantry-room models 1a, 1b shown in the left side.

The same meaning is applied to the arrow in FIG. 16. In FIG. 16, the gantry-room models 1a, 1b in mutually inverted relation are arranged on the right side in the arrangement-region frame 10 so as to be adjacent to each other and in contact with the shield wall 9. Residual spaces 14a, 14b are each a space resulting from subtracting a treatment-room-model related region in which the multiple treatment-room models (gantry-room models 1a, 1b) are arranged, from the target space for arrangement whose boundary is indicated by the arrangement-region frame 10.

The residual space 14a in FIG. 15 is a space partitioned with the shield wall 9 placed between the multiple treatment-room models (gantry-room models 1a, 1b), the outer peripheries of the treatment-room models and the arrangement-region frame 10. The residual space 14b in FIG. 16 is a space which is placed in the left side of a gantry-room-model extended boundary 30 that is provided by extending the left-side outer-periphery line of the gantry-room model 1b that is different to the gantry-room model 1 a in contact with the arrangement-region frame 10, toward the upper side and the lower side of the arrangement-region frame 10.

In the space in the right side of the gantry-room-model extended boundary 30, some spaces are left in the upper side of the gantry-room model 1b, the lower side of the gantry-room model 1a, and the like; however, in each of these spaces, the shield wall 9 is to be placed. Thus, they are not effectively-usable spaces and are thus not assumed as residual spaces.

The two-dimensional region of the treatment-room-model related region is a region which includes: the multiple treatment- models (gantry-room models 1a, 1b); and a region partitioned with the shield wall 9 placed between the multiple treatment-models (gantry-room models 1a, 1b), the outer peripheries of the treatment-room models and the arrangement-region frame 10, or the two-dimensional region is an outside region in which no treatment-room model exists and which is placed outside the treatment-room-model extended boundary (gantry-room-model extended boundary 30) that is provided by extending the outer-periphery line of the treatment-room model toward the arrangement-region frame 10.

The three-dimensional region of the treatment-room-model related region is a three-dimensional region resulting from expanding the two-dimensional region of the treatment-room-model related region vertically from the floor to the ceiling. The two-dimensional region of the residual space 14b in FIG. 16 corresponds to the above outside region.

The concave region 15a with respect to the residual space 14a in FIG. 15 and a concave region 15b with respect to the residual space 14b in FIG. 16 are defined similarly to the concave region 8 described in Embodiment 1. However, any two points $p_1$, $p_2$ are points both in the residual space 14a or the residual space 14b.

Although the residual-space concave regions 15a, 15b are defined similarly to in Embodiment 1, these regions can be defined in other words as follows: the residual-space concave region 15a is a region composed of a set of points on lines that connect to each other, mutually-facing respective points on the outer periphery (outer-periphery points) of the residual space 14a that are placed toward the treatment-room-model related region; likewise, the residual-space concave region 15b is a region composed of a set of points on lines that connect to each other, mutually-facing respective points on the outer periphery (outer-periphery points) of the residual space 14b that are placed toward the treatment-room-model related region. Note that, for the residual spaces, numeral 14 is used collectively, and numerals 14a, 14b are used when they are to be described distinctively. For the residual-space concave regions, numeral 15 is used collectively, and numerals 15a, 15b are used when they are to be described distinctively.

It is desired that the residual space 14 be composed of a convex set. For example, the two-dimensional region of the residual space 14 is easier to use when it is in a quadrangular shape than when it is in L-like shape, even on the same area basis. This is because a quadrangular shape is composed of a convex set, whereas L-like shape has a concave portion and is thus not composed of a convex set. The method of assisting designing of a particle beam therapy facility of Embodiment 3 is an example in which the gantry-room model 1 is displaced according to an operator's instruction, and at each displacement, the volumes or projected areas of the concave region 8 and the residual-space concave region 15 are calculated.

The flowchart of FIG. 13 will be described. The flowchart of FIG. 13 differs from that of FIG. 1 in that Step S012 and Step S013 are added in place of Steps S004 in FIG. 1. Steps S012and S013 that are steps different to those in FIG.

1 will be described.

In Step S012, the computer 25 determines the residual space 14 by subtracting the treatment-room-model related region in which the treatment-room models (gantry-room models 1a, 1b) are arranged, from the arrangement-region frame 10 indicative of the target space for arrangement, and calculates the volume of a concave region with respect to the residual space 14 (residual-space concave region 15) or the area developed when that concave region is two-dimensionally projected (projected area) (residual-space concave region calculation step). In Step S013, the volumes or projected areas calculated in the local-concave region calculation step and the residual-space concave region calculation step are displayed on the display device 26 (concave-region calculation- result display step).

For example, when a calculation start command for the local-concave region calculation step corresponding to Step S003 is input, the local-concave region calculation step and the residual-space concave region calculation step are executed. As shown in FIG. 14, the result from execution of the local-concave region calculation step, the residual-space concave region calculation step and the concave-region calculation-result display step is displayed on the display device 26.

In FIG. 14, there are shown two local concave regions, in which a local concave region 8a is illustrated in the side of the open-space region 2 and a local concave region 8b is illustrated in the side of the gantry body 4. In a residual-space display 29, the residual space 14 and the residual-space concave region 15 are displayed. A concave-region indication 28a represents the volume or projected area of the local concave region 8a, and a concave-region indication 28b represents the volume or projected area of the local concave region 8b. A concave-region indication 28c represents the volume or projected area of the residual-space concave region 15.

Arrangements of the gantry-room models 1a, 1b shown in FIG. 15 and FIG. 16 each show a difference from their initial positions. Because the optimum value is to be found out according to the displacement instruction by the operator, the gantry-room models 1a, 1b generally reach from their initial positions shown in FIG. 14 to the arrangement of the gantry-room models 1a, 1b in FIG. 15.

In order to achieve the arrangement of the gantry-room models 1a, 1b shown in FIG. 16, it suffices to execute the operation after mutually inverting the initial positions of the gantry-room models 1a, 1b, or to continue the operation by inverting one of the gantry-room models in Step S006.

According to the method of assisting designing of a particle beam therapy facility of Embodiment 3, the local concave region 8 and the residual-space concave region 15 are calculated, the arrangement of the treatment-room models (gantry-room models 1a, 1b) is displayed, the local concave region 8 and the residual- space concave region 15 are displayed, and the calculation result of the volumes or projected areas of the local concave region 8 and the residual-space concave region 15 are displayed.

Using the method of assisting designing of a particle beam therapy facility in Embodiment 3 makes it possible to properly arrange the treatment-room models (gantry-room models 1a, 1b) in the arrangement-region frame 10. Thus, the layout of the multiple treatment rooms (gantry rooms 20a, 20b) can be optimized so as to reduce, as much as possible, the concave region 8 that is a useless region and the residual-space concave region 15 with respect to the residual space 14 in which no treatment-room model is arranged.


Embodiment 4

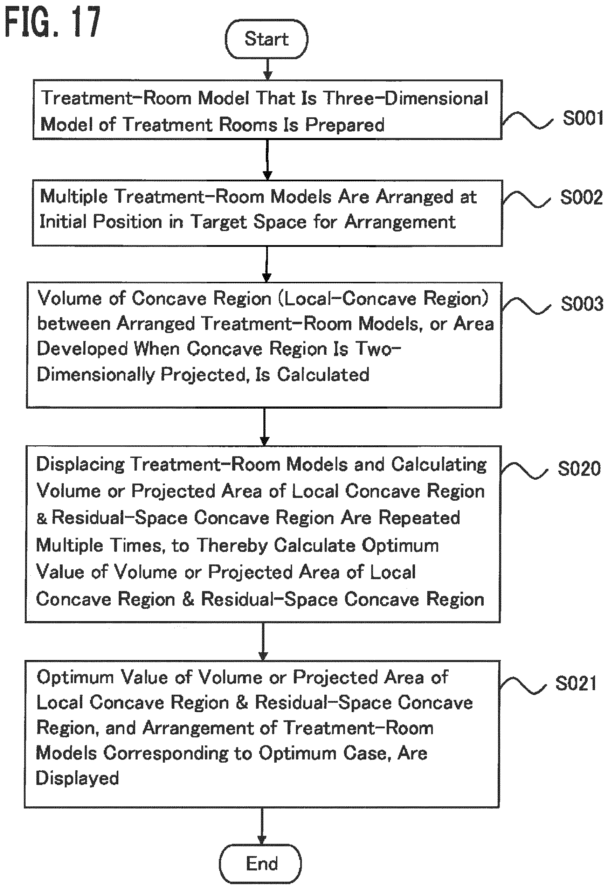

FIG. 17 is a flowchart showing a method of assisting designing of a particle beam therapy facility, according to Embodiment 4 of the invention. In Embodiment 3, an example is shown in which the gantry-room model 1 is displaced according to an operator's instruction, and at each displacement, the volumes or projected areas of the concave region 8 and the residual-space concave region 15 are calculated, whereas what is shown in Embodiment 4 is an example in which their optimum values are calculated through recursive calculation by the computer 25. The flowchart of FIG. 17 differs from that of FIG. 13 in that Step S020 and Step S021 are added in place of Steps S013, S005 and S006 in FIG. 13.

Steps S020 and S021 that are steps different to those in FIG. 13 will be described. In Step S020, the computer 25 repeats multiple times, displacing the gantry-room models 1a, 1b that are the treatment-room models and calculating the volumes or projected areas of the local concave region 8 and the residual-space concave region 15, to thereby calculate optimum values of the volumes or projected areas of the local concave region 8 and the residual-space concave region 15 (optimum-value calculation step).

As a criterion for determining whether the volume or projected area of each of the local concave region 8 and the residual-space concave region 15 is its optimum value or not, the fact is, for example, used that the value of the volume or projected area of each of the local concave region 8 and the residual-space concave region 15 becomes minimum in the arrangement-region frame 10. For example, at the time of recursive calculation of the volume or projected area of each of the local concave region 8 and the residual-space concave region 15, the next position of the treatment-room model is determined using a steepest descent method or the like.

Instead, as a criterion for determining whether the volume or projected area of each of the local concave region 8 and the residual-space concave region 15 is its optimum value or not, the fact may be used that each of evaluation function values weighted on the local concave region 8 and the residual-space concave region 15 becomes minimum. When the local concave region 8 and the residual-space concave region 15 are evaluated after being subjected to weighting, it is possible to more optimize the layout of the multiple treatment rooms (gantry rooms 20a, 20b) than by the method without using weighting.

In Step S021, the optimum values of the volumes or projected regions of the local concave region 8 and the residual- space concave region 15 that have been calculated in Step S020, and the arrangement of the gantry-room models 1a, 1b that are the treatment-room models from which the optimum values have been calculated, are displayed on the display device 26.

It is noted that, when plural similar optimum values (suboptimum values) are present in Step S020, the suboptimum values of the volumes or projected areas of the local concave area 8 and the residual-space concave region 15 corresponding to the extracted suboptimum values, and each arrangement of the gantry-room models 1a, 1b that are the treatment-room models from which the suboptimum values have been calculated, are displayed on the display device 26 in Step S021 (optimum-value calculation-result display step).

With respect to the arrangements of the gantry-room models 1a, 1b shown in FIG. 15 and FIG. 16, though depending on the difference from the initial positions in some cases, when a genetic algorism is applied for changing the arrangement of the gantry-room models 1a, 1b, it is possible to achieve each of the arrangements in FIG. 15 and FIG. 16, according to the optimum values of the volumes or projected areas of the local concave region 8 and the residual-space concave region 15, regardless of the initial positions.

According to the method of assisting designing of a particle beam therapy facility of Embodiment 4, the computer 25 calculates, through recursive calculation, the optimum values of the volumes or projected areas of the concave regions (the local concave region 8 and the residual-space concave region 15), so that the layout of the multiple treatment rooms (gantry rooms 20a, 20b) can be optimized so as to reduce the concave region 8 and the residual- space concave region 15 as much as possible, faster than in the case of Embodiment 3. Further, according to the method of assisting designing of a particle beam therapy facility of Embodiment 4, the layout of the multiple treatment rooms (gantry rooms 20a, 20b) can be optimized more efficiently than in the case of Embodiment 3.

Embodiment 5

FIG. 18 is a flowchart showing a method of constructing a particle beam therapy facility, according to Embodiment 5 of the invention. FIG. 19 is a diagram showing an example in which two treatment-room models and an accelerator model are optimally arranged in the target space for arrangement, and FIG. 20 is a diagram showing an example of a particle beam therapy facility according to Embodiment 5 of the invention. In Embodiment 5, a method of constructing a particle beam therapy facility will be described in which the arrangement of the accelerator is also taken into consideration.

In Step S031, in the arrangement-region frame 10 indicative of the target space for arrangement, an accelerator model 16 is arranged at its initial position (accelerator model arrangement step). In Step S032, in the arrangement-region frame 10, the multiple treatment-room models (gantry-room models 1a, 1b) are arranged at their optimum positions by use of the method of assisting designing of a particle beam therapy facility described in Embodiments 1 to 4 (treatment-room model optimum-arrangement step). In Step S033, in the arrangement-region frame 10, a transport system model 17 that connects the accelerator model 16 with the multiple treatment-room models (gantry-room models 1a, 1b) is arranged (transport-system model arrangement step).

The arrangement-region frame 10 shown in FIG. 19 is in line with the shape of the building in which the particle beam therapy facility 51 is arranged, and is indicated as a broken-line frame in FIG. 20. FIG. 19 shows an arrangement after the completion by the operation in the flowchart in FIG. 18. The particle beam therapy facility 70 constructed based on the arrangement of FIG. 19 is shown in FIG. 20.

In the particle beam therapy facility 70 in FIG. 20, two gantry rooms 20a, 20b and an accelerator room 71 that are partitioned with a shield wall 19, are provided in the left-side region of the building wall 21. In the accelerator room 71, the accelerator 54 and the pre-accelerator 53 are arranged, and in the gantry rooms 20a, 20b, the rotary gantries 24a, 24b are arranged, respectively.

The beam transport system 59 is arranged astride between the gantry rooms 20a, 20b and the accelerator room 71. In the particle beam therapy facility 70 in FIG. 20, relative positions among the two gantry rooms 20a, 20b, the accelerator 54 and the beam transport system 59 are the same as those among the gantry bodies 4 of the gantry-room models 1a, 1b, the accelerator model 16 and the transport system model 17 in FIG. 19.

The arrangement of the gantry-room models 1a, 1b shown in FIG. 19 corresponds to the arrangement example illustrated in FIG. 8. As described in Embodiment 1, the projected area of the concave regions 8 between the treatment-room models, namely, the projected area of a total region of the concave regions 8a, 8b, is equal to or less than one fourth of the projected area of the gantry-room model that is the gantry-room model 1a or the gantry-room model 1b.

Accordingly, the projected area of local concave regions 73 between model-corresponding frames 72a, 72b that correspond to the boundaries of the gantry-room models 1a, 1b in the particle beam therapy facility 70 shown in FIG. 20, namely, the projected area of a total region of four local concave regions 73 shown in FIG. 20, is equal to or less than one fourth of the projected area of the model-corresponding frame 72a or the model-corresponding frame 72b.

The region surrounded by each of the model-corresponding frames 72a, 72b is the virtual gantry region. In the virtual gantry region, the open-space region 2 of the gantry-room model 1 is an open-space region, and a region of the gantry-room model 1 other than the open-space region 2, namely, the region including the gantry body 4 and the gantry front panel 3 of the gantry-room model 1 is a gantry region.

According to the method of constructing a particle beam therapy facility of Embodiment 5, at the time of constructing the particle beam therapy facility 70 which includes the multiple gantry rooms 20a, 20b in which the rotary gantries 24a, 24b are respectively provided, the accelerator 54 and the beam transport system 59, the method of assisting designing of a particle beam therapy facility described in Embodiments 1 to 4 is used with respect to the arrangement of the multiple gantry rooms 20a, 20b.

Thus, it is possible to properly arrange the gantry-room models 1a, 1b in the arrangement-region frame 10, so that the layout of the multiple treatment rooms (gantry rooms 20a, 20b) can be optimized so as to reduce, as much as possible, the local concave region 8 that is a useless region and the residual-space concave region 15. Further, according to the method of constructing a particle beam therapy facility of Embodiment 5, the layout is studied using the arrangement-region frame 10 with a shape matched to the shape of the building in which the gantry rooms 20a, 20b are to be placed, so that the shape and size of each of the treatment rooms, such as the gantry rooms 20a, 20b and the like, and the shape and size of the installation place for the treatment rooms, can be taken into consideration.

The method of constructing a particle beam therapy facility of Embodiment 5 comprises: the accelerator model arrangement step of arranging the accelerator model 16 corresponding to the accelerator 54 in the model space corresponding to the target space for arrangement; the treatment-room model optimum-arrangement step of arranging, in the model space, the multiple treatment-room models (gantry-room models 1a, 1b) that are three-dimensional models of the treatment rooms (gantry rooms 20a, 20b); and the transport- system model arrangement step of arranging, in the model space, the transport system model 17 corresponding to the beam transport system 59 so that it connects the acceleration model 16 with the treatment-room models (gantry-room models 1a, 1b).

In the treatment- room model optimum-arrangement step, the arrangement of the treatment-room models (gantry-room models 1a, 1b) is determined using the method of assisting designing of a particle beam therapy facility shown in Embodiments 1 to 4.

Because of this configuration, according to the method of constructing a particle beam therapy facility of Embodiment 5, the layout is studied using the arrangement-region frame 10 with a shape matched to the shape of the building in which the multiple treatment rooms (gantry rooms 20a, 20b) are to be placed, so that it is possible to construct the particle beam therapy facility in which the shape and size of each of the treatment rooms such as the gantry rooms 20a, 20b and the like, and the shape and size of the installation place for the treatment rooms, are taken into consideration.

The particle beam therapy facility of Embodiment 5 comprises: the beam generation apparatus 52 for generating the charged particle beam 31 and accelerating the charged particle beam 31 using the accelerator 54; the beam transport system 59 for transporting the charged particle beam 31 accelerated by the accelerator 54; the multiple particle beam irradiation apparatuses 58a, 58b each for radiating the charged particle beam 31 transported by the beam transport system 59 to an irradiation target (diseased site 48); the multiple rotary gantries 24a, 24b equipped respectively with the particle beam irradiation apparatuses 58a, 58b, each for radiating the charged particle beam 31 to the irradiation target (diseased site 48) from an arbitrary direction; and the multiple treatment rooms (gantry rooms 20a,20b) in which the rotary gantries 24a, 24b are respectively placed.

When such a region is defined as a virtual gantry region that is composed of: a gantry region including the body and the front panel of each of the rotary gantries 24a, 24b; and an open-space region in the treatment room (gantry rooms 20a, 20b) that is connected to an inner region of the body of each of the rotary gantries 24a, 24b, and when, with respect to the two treatment rooms (gantry rooms 20a, 20b) that are arranged most adjacent to each other through the shield wall 19, such a region is defined as the local concave region 73 that is composed of a set of object points that are points on lines placed between the two virtual gantry regions and connecting to each other, mutually-facing outer peripheries of the two virtual gantry regions, except for points in the shield wall 19, the multiple treatment rooms (gantry rooms 20a, 20b) are arranged so that a projected area which is developed when the local concave region 73 between the two treatment rooms (gantry rooms 20a, 20b) among the multiple treatment rooms (gantry rooms 20a, 20b), that are arranged most adjacent to each other, is two-dimensionally projected toward the floor, is equal to or less than one fourth of a projected area which is developed when the virtual gantry region is two-dimensionally projected toward the floor. Because of this configuration, according to the particle beam therapy facility of Embodiment 5, the layout of the multiple treatment rooms (gantry rooms 20a, 20b) can be optimized so as to reduce the local concave region 73 as much as possible.

It is noted that, although the multiple gantry-room models have been illustrated as each having the same outer periphery (boundary), each of the gantry-room models is to be prepared in conformity with the actual rotary gantry and thus, if the multiple gantry-room models have different outer peripheries (boundaries), this invention may also be applied thereto.

Further, although the irradiation method of the particle beam irradiation apparatus 58 has been described citing a scanning irradiation method as an example, this invention may also be applied to the particle beam therapy facility 70 which includes the particle beam irradiation apparatus 58 using a broad irradiation method in which the charged particle beam 31 is scattered and enlarged by a scatterer and an irradiation field is formed from the enlarged charged particle beam 31 to be in conformity with an irradiation target 48.

Further, this invention may also be applied to the particle beam therapy facility 70 which includes the particle beam irradiation apparatus 58 using another scanning irradiation method different to the scanning irradiation method described in Embodiment 1, namely, a spot- scanning irradiation method, a raster-scanning irradiation method or the like. Further, unlimited combination of the respective embodiments, and appropriate modification and omission in the embodiments may be made in the present invention without departing from the scope of the invention.

DESCRIPTION OF REFERENCE NUMERALS and SIGNS

| | |
|---|---|
| 1, 1a, 1b | gantry-room model |
| 8, 8a, 8b | concave region (local concave region) |
| 9, 9a, 9b, | shield wall |
| 9c, 9d | shield wall |
| 14, 14a, 14b | residual space |
| 15, 15a, 15b | concave region (residual-space concave region) |
| 16 | accelerator model |
| 17 | transport system model |
| 19, 19a, 19b | shield wall |
| 20a, 20b | gantry room |
| 24a, 24b | rotary gantry |
| 26 | display device |
| 27 | design assisting device |
| 31 | charged particle beam |
| 48 | diseased site (irradiation target) |
| 51 | particle beam therapy system |
| 52 | beam generation apparatus |
| 54 | accelerator |
| 58, 58a, 58b | particle beam irradiation apparatus |
| 59 | beam transport system |
| 73 | concave region. |

**Claims**

1. A method of assisting designing of a particle beam therapy facility, which uses a design assisting device to thereby assist arrangement designing for arranging, in a predetermined target space for arrangement, multiple treatment rooms in which rotary gantries each for radiating a charged particle beam to an irradiation target from an arbitrary direction are to be placed,
the method of assisting designing of a particle beam therapy facility comprising:

- a treatment-room model preparation step of preparing a treatment-room model that is a three-dimensional model of each of the treatment rooms;
- a treatment-room model arrangement step of arranging multiple treatment-room models each being the treatment-room model, at initial positions in a model space corresponding to the target space for arrangement;
- a local-concave region calculation step of calculating: a volume of a local concave region which is a concave region between two the treatment-room models among the multiple treatment-room models, that are arranged most adjacent to each other; or a projected area which is developed when the local concave region is two-dimensionally projected toward a floor;
- a concave-region calculation-result display step of displaying the volume or the projected area of the local concave region calculated in the local-concave region calculation step, on a display device of the design assisting device; and

- a treatment-room model displacement step of displacing the treatment-room model in the model space in response to a displacement instruction for that treatment-room model, when an operation- termination instruction is not issued after the concave-region calculation-result display step;
- wherein the local-concave region calculation step, the concave-region calculation-result display step and the treatment- room model displacement step are repeated until the operation- termination instruction is issued; and
- wherein the local concave region is a region composed of a set of points on lines that connect to each other, mutually-facing outer peripheries of the two treatment-room models arranged most adjacent, or, in a case where a shield wall is placed between the two treatment-room models arranged most adjacent, a region composed of a set of object points that are points on lines that connect to each other, mutually-facing outer peripheries of the two treatment-room models in that case, except for points in the shield wall.

2. A method of assisting designing of a particle beam therapy facility, which uses a design assisting device to thereby assist arrangement designing for arranging, in a predetermined target space for arrangement, multiple treatment rooms in which rotary gantries each for radiating a charged particle beam to an irradiation target from an arbitrary direction are to be placed,
the method of assisting designing of a particle beam therapy facility comprising:

- a treatment-room model preparation step of preparing a treatment-room model that is a three-dimensional model of each of the treatment rooms;
- a treatment-room model arrangement step of arranging multiple treatment-room models each being the treatment-room model, at initial positions in a model space corresponding to the target space for arrangement;
- a local-concave region calculation step of determining, among the multiple treatment-room models, each two of the treatment-room models that are arranged most adjacent to each other, as a target pair, and calculating, for each target pair: a volume of a local concave region which is a concave region between two the treatment-room models in the target pair; or a projected area which is developed when the local concave region is two-dimensionally projected toward a floor;
- an optimum-value calculation step of repetitively performing, multiple times for the each target pair, displacing the treatment- room model by use of the design assisting device and calculating the volume of the local concave region or the projected area of the local concave region, to thereby calculate an optimum value thereof; and
- an optimum-value calculation-result display step of displaying the optimum value of the volume of the local concave region or the optimum value of the projected area that is calculated in the optimum-value calculation step, and an arrangement of the treatment-room models corresponding to a case of that optimum value, on a display device of the design assisting device;
- wherein the local concave region is a region composed of a set of points on lines that connect to each other, mutually-facing outer peripheries of the two treatment-room models arranged most adjacent, or, in a case where a shield wall is placed between the two treatment-room models arranged most adjacent, a region composed of a set of object points that are points on lines that connect to each other, mutually-facing outer peripheries of the two treatment-room models in that case, except for points in the shield wall.

3. The method of assisting designing of a particle beam therapy facility according to claim 1,
further comprising a residual-space concave region calculation step of determining a residual space by subtracting, from the model space, a treatment-room-model related region including the multiple treatment-room models, and then calculating: a volume of a residual-space concave region which is a concave region with respect to the residual space; or a projected area which is developed when the residual-space concave region is two-dimensionally projected toward a floor;

- wherein the residual-space concave region is a region composed of a set of points on lines that connect to each other, mutually- facing outer periphery portions of the residual space that are placed toward the treatment-room-model related region; and
- wherein, in the concave-region calculation-result display step, a calculation result by the local-concave region calculation step and the volume or the projected area of the residual-space concave region calculated in the residual-space concave region calculation step, are displayed on the display device.

4. The method of assisting designing of a particle beam therapy facility according to claim 2,
further comprising a residual-space concave region calculation step of determining a residual space by subtracting, from the model space, a treatment-room-model related region including the multiple treatment-room models, and then calculating: a volume of a residual-space concave region which is a concave region with respect to the residual space; or a projected area which is developed when the residual-space concave region is two-dimensionally projected toward a floor;

- wherein the residual-space concave region is a region composed of a set of points on lines that connect to each other, mutually-facing outer periphery portions of the residual space that are placed toward the treatment-room-model related region;
- wherein, in the optimum-value calculation step, calculation of the volume of the local concave region or the projected area of the local concave region, and calculation of the volume of the residual-space concave region or the projected area of the residual- space concave region, at the time the treatment-room model is displaced by use of the design assisting device, are repeated multiple times, for the each target pair, to thereby calculate optimum values thereof; and
- wherein, in the optimum-value calculation-result display step, the optimum value of the volume of the local concave region or the optimum value of the projected area thereof calculated in the optimum-value calculation step, the optimum value of the volume of the residual-space concave region or the optimum value of the projected area thereof, and an arrangement of the treatment-room models corresponding to a case of these optimum values, are displayed on the display device of the design assisting device.

5. The method of assisting designing of a particle beam therapy facility according to claim 4,
wherein, in the optimum-value calculation step, the optimum value of the volume of the local concave region or the optimum value of the projected area thereof and the optimum value of the volume of the residual-space concave region or the optimum value of the projected area thereof, are calculated based on evaluation function values which are weighted according to a relationship between the local concave region and the residual-space concave region.

6. A method of constructing a particle beam therapy facility by arranging, in a predetermined target space for arrangement, an accelerator for a particle beam therapy system, a beam transport system for transporting a charged particle beam accelerated by the accelerator and multiple treatment rooms in which rotary gantries each for radiating the charged particle beam to an irradiation target from an arbitrary direction are to be placed,
the method of constructing a particle beam therapy facility comprising:

- an accelerator model arrangement step of arranging an accelerator model corresponding to the accelerator in a model space corresponding to the target space for arrangement;
- a treatment-room model optimum-arrangement step of arranging, in the model space, multiple treatment-room models that are three- dimensional models of the treatment rooms; and
- a transport-system model arrangement step of arranging, in the model space, a transport system model corresponding to the beam transport system so that it connects the acceleration model with the treatment-room models;

wherein, in the treatment-room model optimum-arrangement step, an arrangement of the treatment-room models is determined using the method of assisting designing of a particle beam therapy facility according to any one of claims 1 to 5.

7. The method of constructing a particle beam therapy facility according to Claim 6,
wherein, in the treatment-room model optimum-arrangement step, the treatment-room models are arranged so that the projected area of the local concave region is equal to or less than one fourth of a projected area of the treatment-room model.

8. A particle beam therapy facility, comprising:

- a beam generation apparatus for generating a charged particle beam and accelerating the charged particle beam using an accelerator;
- a beam transport system for transporting the charged particle beam accelerated by the accelerator; multiple particle beam irradiation apparatuses each for radiating the charged particle beam transported by the beam transport system to an irradiation target;
- multiple rotary gantries equipped respectively with the particle beam irradiation apparatuses, each for radiating the charged particle beam to the irradiation target from an arbitrary direction; and
- multiple treatment rooms in which the rotary gantries are respectively placed; wherein,

when such a region is defined as a virtual gantry region that is composed of: a gantry region including a body and a front panel of each of the rotary gantries; and an open-space region in the treatment room that is connected to an inner region of the body of each of the rotary gantries, and

when, with respect to two the treatment rooms that are arranged most adjacent to each other through a shield wall, such a region is defined as a local concave region that is composed of a set of object points that are points on lines placed between two virtual gantry regions each being the virtual gantry region and connecting to each other, mutually-facing outer peripheries of the two virtual gantry regions, except for points in the shield wall,

the multiple treatment rooms are arranged so that a projected area which is developed when the local concave region between the two treatment rooms among the multiple treatment rooms, that are arranged most adjacent to each other, is two-dimensionally projected toward a floor, is equal to or less than one fourth of a projected area which is developed when the virtual gantry region is two-dimensionally projected toward the floor.

EP 3 222 321 A1

# FIG. 1

```
                        ┌─────────┐
                        │  Start  │
                        └─────────┘
                             │
                             ▼
  ┌──────────────────────────────────────────────┐
  │ Treatment-Room Model That Is Three-Dimensional │  ⟩~ S001
  │ Model of Treatment Rooms Is Prepared           │
  └──────────────────────────────────────────────┘
                             │
                             ▼
  ┌──────────────────────────────────────────────┐
  │ Multiple Treatment-Room Models Are Arranged at │  ⟩~ S002
  │ Initial Position in Target Space for Arrangement│
  └──────────────────────────────────────────────┘
                             │
       ┌─────────────────────┼
       │                     ▼
  ┌──────────────────────────────────────────────┐
  │ Volume of Concave Region (Local-Concave Region)│  ⟩~ S003
  │ between Arranged Treatment-Room Models, or Area│
  │ Developed When Concave Region Is Two-          │
  │ Dimensionally Projected, Is Calculated          │
  └──────────────────────────────────────────────┘
                             │
                             ▼
  ┌──────────────────────────────────────────────┐
  │ Volume or Projected Area of Local-Concave Region│  ⟩~ S004
  │ Is Displayed                                    │
  └──────────────────────────────────────────────┘
                             │
                             ▼        S005
                        ◇─────────────────◇
       ◄────────  Is Assisting Operation Completed?  ────────► ┌─────┐
                        ◇─────────────────◇         Yes        │ End │
                             │                                  └─────┘
                             │ No
                             ▼
  ┌──────────────────────────────────────────────┐
  │ Treatment-Room Model Is Displaced to Be Re-    │  ⟩~ S006
  │ Arranged in Response to Displacement Instruction│
  └──────────────────────────────────────────────┘
       └─────────────────────┘
```

18

FIG. 2

FIG. 3

# FIG. 4

FIG. 5

51

59

46            47

55e

55f

55b

55a

54    53

55d      56b      52

56a          58b

55c    58a

EP 3 222 321 A1

# FIG. 6

FIG. 7

FIG. 8

FIG. 9

10

1a

9

1b

FIG. 10

70

21

18a

24a

72a

20a

19b    18b    72b    20b    24b    19a

FIG. 11

# FIG. 12

Start

Treatment-Room Model That Is Three-Dimensional Model of Treatment Rooms Is Prepared — S001

Multiple Treatment-Room Models Are Arranged at Initial Position in Target Space for Arrangement — S002

Volume of Concave Region (Local-Concave Region) between Arranged Treatment-Room Models, or Area Developed When Concave Region Is Two-Dimensionally Projected, Is Calculated — S003

Displacing Treatment-Room Models and Calculating Volume or Projected Area of Local Concave Region Are Repeated Multiple Times, to Thereby Calculate Optimum Value of Volume or Projected Area of Local Concave Region — S010

Optimum Value of Volume or Projected Area of Local Concave Region, and Arrangement of Treatment-Room Models Corresponding to Optimum Case, Are Displayed — S011

End

# FIG. 13

$$\boxed{\text{Start}}$$

Treatment-Room Model That Is Three-Dimensional Model of Treatment Rooms Is Prepared   S001

Multiple Treatment-Room Models Are Arranged at Initial Position in Target Space for Arrangement   S002

Volume of Concave Region (Local-Concave Region) between Arranged Treatment-Room Models, or Area Developed When Concave Region Is Two-Dimensionally Projected, Is Calculated   S003

Residual Space Is Determined by Subtracting Treatment-Room-Model Related Region in Which Treatment-Room Models Are Arranged, from Target Space for Arrangement, and
Volume of Concave Region with Respect to Residual Space (Residual-Space Concave Region) or Area Developed When Concave Region Is Two-Dimensionally Projected, Is Calculated   S012

Volumes or Projected Areas of Local Concave Region and Residual-Space Concave Region Are Displayed   S013

Is Assisting Operation Completed?   S005

Yes → End

No

Treatment-Room Model Is Displaced to Be Re-Arranged in Response to Displacement Instruction   S006

27

FIG. 14

1b   8a   1a   26

28a
A: xxxa

28b
B: xxxb

10

8b   9

28c
C: xxxc

14

15

29

FIG. 15

14a   1a   14a

1b   9   10   15a

EP 3 222 321 A1

# FIG. 16

29

# FIG. 17

Start

Treatment-Room Model That Is Three-Dimensional Model of Treatment Rooms Is Prepared — S001

Multiple Treatment-Room Models Are Arranged at Initial Position in Target Space for Arrangement — S002

Volume of Concave Region (Local-Concave Region) between Arranged Treatment-Room Models, or Area Developed When Concave Region Is Two-Dimensionally Projected, Is Calculated — S003

Displacing Treatment-Room Models and Calculating Volume or Projected Area of Local Concave Region & Residual-Space Concave Region Are Repeated Multiple Times, to Thereby Calculate Optimum Value of Volume or Projected Area of Local Concave Region & Residual-Space Concave Region — S020

Optimum Value of Volume or Projected Area of Local Concave Region & Residual-Space Concave Region, and Arrangement of Treatment-Room Models Corresponding to Optimum Case, Are Displayed — S021

End

# FIG. 18

Start

Accelerator Model Is Arranged in Target Space for Arrangement — S031

Treatment-Room Models Are Arranged in Target Space for Arrangement — S032

Transport System Model That Connects Accelerator Model with Treatment-Room Models Is Arranged in Target Space for Arrangement — S033

End

# FIG. 19

# FIG. 20

## INTERNATIONAL SEARCH REPORT

| International application No. |
|---|
| PCT/JP2014/080899 |

### A. CLASSIFICATION OF SUBJECT MATTER

$A61N5/10$(2006.01)i, $E04H3/08$(2006.01)i, $G06F17/50$(2006.01)i

According to International Patent Classification (IPC) or to both national classification and IPC

### B. FIELDS SEARCHED

Minimum documentation searched (classification system followed by classification symbols)
A61N5/10, E04H3/08, G06F17/50

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2011-092424 A (Sumitomo Heavy Industries, Ltd.), 12 May 2011 (12.05.2011), entire text; all drawings & US 2011/0101246 A1 & EP 2317522 A1 & CN 102049104 A & TW 201118888 A & HK 1157265 A | 1,3,6,7 |
| A | JP 2009-502221 A (GSI Gesellschaft für Schwerionenforschung mbH), 29 January 2009 (29.01.2009), entire text; all drawings & US 2008/0203323 A1 & EP 1909905 A & WO 2007/009786 A1 & DE 102005035141 A & AT 502674 T | 1,3,6,7 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 February 2015 (24.02.15) | 10 March 2015 (10.03.15) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (July 2009)

**INTERNATIONAL SEARCH REPORT**

International application No.

PCT/JP2014/080899

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-528659 A (GSI Gesellschaft für Schwerionenforschung mbH), 30 September 2003 (30.09.2003), entire text; all drawings & US 2003/0141460 A1 & EP 1261394 A & WO 2001/066187 A1 & DE 10010523 A & BR 108976 A & AT 303843 T & CN 1411385 A | 1,3,6,7 |
| A | JP 2001-522098 A (Dr. Baldeweg GmbH), 13 November 2001 (13.11.2001), entire text; all drawings & US 6734847 B1 & EP 1025520 A & WO 1999/023586 A2 & DE 19747881 A | 1,3,6,7 |
| A | JP 01-121972 A (Toshiba Corp.), 15 May 1989 (15.05.1989), entire text; all drawings (Family: none) | 1,3,6,7 |

Form PCT/ISA/210 (continuation of second sheet) (July 2009)

## INTERNATIONAL SEARCH REPORT

| International application No. |
| --- |
| PCT/JP2014/080899 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
     The inventions of claims 1, 3, 6, and 7 relate to a particle therapy facility design support method for allowing a design support system to support design of location in which a local recessed region computation procedure, a recessed region result display procedure, and a treatment chamber model relocation procedure are repeated until a work end command is issued, whereby a plurality of treatment chambers in which a rotational gantry is set up to irradiate a subject to be irradiated with a charged particle beam in an arbitrary direction are located in a space at a predetermined location of interest.
(Continued to extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.    Consequently, this international search report is restricted to the invention first mentioned in the claims; it is covered by claims Nos.:
   1, 3, 6 and 7

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2014/080899

Continuation of Box No.III of continuation of first sheet(2)

The inventions of claims 2, and 4 to 7 relate to a particle therapy facility design support method, the method being adapted for a set of two treatment chamber models of interest that are located closest to each other and including, for each set of interest, the local recessed region computation procedure, an optimum value computation procedure, and an optimum value computation result display procedure.

The invention of claim 8 relates to a particle therapy facility wherein a plurality of treatment chambers are located so that a projected area given when a local recessed region between two treatment chambers disposed to be closest to each other among the plurality of treatment chambers is projected onto a two-dimensional plane in the direction of the floor is 1/4 or less than a projected area given when an imaginary gantry region is projected onto a two-dimensional plane in the direction of the floor.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012100915 A
- JP 5526166 B